# EUROPÄISCHE PATENTANMELDUNG

(11) **EP 0 567 887 A2**
(43) Veröffentlichungstag der Anmeldung: **03.11.1993**
(21) Anmeldenummer: 93106333.3
(22) Anmeldetag: 20.04.1993
(51) Int. Cl.: C12P 1/00, C12P 21/02, C07K 5/06, C12M 1/40, C07K 5/08

(54) **Enzymatische Reaktionen und Verwendung einer Vorrichtung zu ihrer Durchführung**

(30) Priorität: 29.04.1992 DE 4214157
(71) Anmelder: Degussa Aktiengesellschaft, D-60311 Frankfurt (DE)
(72) Erfinder: Kuhl, Peter, Dr., O-7280 Eilenburg (DE); Eichhorn, Uwe, O-8101 Grosserkammsdorf (DE); Jakubke, Hans-Dieter, Prof. Dr., O-7030 Leipzig (DE); Drauz, Karlheinz, Prof. Dr., W-6463 Freigericht 1 (DE); Bommarius, Andreas, Dr., W-6000 Frankfurt am Main (DE)

(57) **Zusammenfassung**

Wasserunterstützte enzymatische Reaktion, wobei das Substrat und das Enzym in fester Phase vorliegen und wobei entweder das Wasser kein flüssiges Kontinuum um Substrat und Enzym bildet, oder das Wasser zumindest zum Teil aus der Gasphase wirkt, oder das für Reaktion notwendige Wasser über einen Festkörper zugesetzt wird, oder zumindest ein Teil des für die Reaktion notwendigen Wassers durch Festkörperkontakte übertragen wird, oder eine Kombination der Möglichkeiten. Das Reaktionsgemisch kann durch Ultraschall oder Verwirbelung, z.B. in einer Wirbelschicht, vermischt werden.

## Beschreibung

Die Erfindung betrifft wasserunterstützte enzymatische Reaktionen zwischen einem Substrat und einem Enzym gemäß dem Oberbegriff des Anspruches 1 bzw. 4 sowie die Verwendung einer Vorrichtung zur Durchführung einer enzymatischen Reaktion.

Vorzugsweise kommt das erfindungsgemäße Verfahren bei Reaktionen zum Einsatz, die in wäßrigen Systemen wegen Hydrolyse der Edukte oder wegen ungünstiger Lage eines Gleichgewichts unter Beteiligung von Wasser nicht oder nur schlecht durchgeführt werden können.

Enzymatische Reaktionen sind grundsätzlich wasserunterstützt, d. h. das Substrat und das Enzym benötigen Wasser, um miteinander reagieren zu können. Im vorliegenden ist dabei "einem" nicht limitierend zu verstehen, enzymatische Reaktionen der eingangs genannten Art können Umsetzungen von einem einzigen oder mehreren Substrat/en unter Verwendung eines einzigen oder mehreren Enzym/en sein, wobei zusätzlich noch weitere Stoffe, wie z. B. Cofaktoren, zugegen sein können. Unter Substrat ist also im folgenden ein Edukt zu verstehen, das zum gewünschten Produkt umgesetzt wird.

Es sind bereits Reaktionssysteme bekannt, in denen der Wassergehalt gesenkt wird, um Hydrolysereaktionen zu unterdrücken oder das Gleichgewicht in Richtung auf die Wasser enthaltene Seite zu verschieben. So wird z. B. mit organischen Lösungsmitteln gearbeitet, denen, je nach Löslichkeit, mehr oder weniger Wasser zugeführt wird (siehe z. B.: A. M. Klibanov, Trends in Biochem. Sci. 1989, Vol. 14, 141 - 44). Auch sind Enzymreaktionen in inversen Mizellmedien durchgeführt worden (J. W. Shield, H. D. Ferguson, A. S. Bommarius und T. A. Hatton, Ind. Eng. Chem. 1986, Vol. 25, 603 - 12).

Ferner wurden Reaktionen in der Gasphase durchgeführt, z. B. mit gasförmigen Alkoholen als Substrat (E. Barzana, M. Karel und A. M. Klibanov, Biotech. Bioeng. 1989, Vol. 34, 1178 - 85; S. Pulvin, F. Parvaresh, D. Thomas und M. D. Legoy, Ann. N. Y. Acad. Sci. 1988, Vol. 542, 434 - 9) oder mit gasförmigen Estern und Alkoholen zur Umesterung (F. Parvaresh, H. Robert, D. Thomas und M. D. Legoy, Biotech. Bioeng. 1992, Vol. 39, 467 - 73).

Bei den obengenannten Reaktionen wurden teils neben dem immobilisierten Enzym Cofaktoren verwendet, die nicht regeneriert und/oder auf festen Trägern, die mit Wasser unter einem Partialdruck gesättigt waren, immobilisiert wurden.

Enzymatische Reaktionen in einem organischen Lösungsmittel haben den Nachteil, daß es häufig zu Wechselwirkungen zwischen dem Enzym und dem organischen Lösungsmittel kommt. Diese Wechselwirkungen zwischen dem organischen Lösungsmittel und dem Enzym führen dabei häufig zu Veränderungen der Struktur des Enzyms mit ungünstigen Auswirkungen auf seine Stabilität und Aktivität, wodurch bei Peptidsynthesereaktionen die Peptidbindungsknüpfung beeinträchtigt wird (JAKUBKE, H.-D., KUHL, P., KÖNNECKE, A.: Angew. Chem. 97, 79 - 87 (1985); Angew. Chem. Int. Ed. Engl. 24, 85 (1985); JAKUBKE, H.-D., in: "The Peptides: Analysis, Synthesis, Biology" (UDENFRIEND, S., MEIENHOFER, J., Eds.) Academic Press, New York (1987), 103 - 165; KULLMANN, W.: "Enzymatic Peptide Synthesis", CRC Press, Boca Raton (1987); JAKUBKE, H.-D., Kontakte 1991 (3), 60; ibid. 1992 (1), 46).

Reaktionen in der Gasphase sind schwer kontrollierbar, wobei das gasförmige Substrat außerdem wie ein organisches Lösungsmittel Wechselwirkungen mit dem Enzym eingehen kann. Aus diesem Grund sind solche Enzymreaktionen auf wenige Substrate und Enzyme beschränkt und großtechnisch meist nicht durchführbar.

Seit kurzem sind auch Reaktionen in der festen Phase bekannt, bei denen das Substrat und das Enzym in Gegenwart von Na₂CO₃·10H₂O verrieben werden. Das Hydratwasser des Salzes dient dabei zur Versorgung des Reaktionsgemisches mit der erforderlichen Wassermenge. Das Vermischen erfolgt mittels eines Homogenisators. Ein Scale-up ist hier problematisch, da die innige Vermischung nicht mehr so ohne weiteres mittels eines Homogenisators sichergestellt werden kann. Außerdem kann je nach Synthese die Gegenwart hoher Salzkonzentrationen störend und die anschließende Aufarbeitung problematisch sein.

Aufgabe der vorliegenden Erfindung ist daher ein möglichst universell durchführbares enzymatisches Verfahren zur Herstellung von Substraten, bei dem hydrolytische Nebenreaktionen, organische Lösungsmittel und gewünschtenfalls auch hohe Salzkonzentrationen weitgehend vermieden werden können, wodurch meist eine vereinfachte und umweltverträglichere Prozeßführung möglich sein soll.

Bei einer wasserunterstützten enzymatischen Reaktion der eingangs beschriebenen Art wird diese Aufgabe mit den Maßnahmen der Ansprüche 1 oder 4 gelöst.

All diesen Maßnahmen ist gemeinsam, daß das Substrat und das Enzym in fester Phase vorliegen, und daß möglichst mit einer minimalen Wassermenge gearbeitet wird, die so bemessen ist, daß lediglich eine Enzymaktivität gegeben ist, aber eventuelle Nebenreaktionen wie Hydrolyse weitgehend unterdrückt sind. Im Regelfall kann dabei auf organische Lösungsmittel verzichtet werden. Bei einer solch geringen Wassermenge werden Enzym und Substrat nicht gelöst, sondern nur hydratisiert, so daß diese als Festkörper einander in Kontakt gebracht werden müssen. Bei gewöhnlichen Enzymreaktionen ist zumindest ein Partner, meist das Substrat, in einem fluiden, d. h. flüssigen oder gasförmigen Zustand, so daß ein Kontakt zur Durchführung der Reaktion schon allein durch Konvektionsbewegung der fluiden Phasen gegeben ist.

Im vorliegenden Fall wird wegen der fehlenden fluiden Phase das Substrat-Enzymgemisch mechanisch bewegt und dabei in innigen Kontakt zueinander gebracht. Diese Bewegung geschieht je nach Verfahren durch Anlegen von Ultraschall (ausreichender Energie) oder durch Aufwirbelung durch Durchleiten eines Gases, wobei eine Wirbelschicht besonders bevorzugt ist.

Enzymatische Umsetzungen, bei denen feuchte Luft durch ein Substrat hindurchgeleitet und ggf. eine Wirbelschicht erzeugt wird, sind aus Feststoff-Fermentationen (z. B. DE-A 32 37 896, "forum mikrobiologie" 9/88, S. 386 - 394) bekannt. Die Feststoff-Fermentation, die in einigen mikrobiologischen Prozessen Vorteile gegenüber der "Stirred Tank Fermentation" hat, benötigt große Mengen an Luft und Feuchtigkeit, um einerseits die mikrobiologischen Prozesse in Gang zu halten und andererseits die entstehende Wärme abzuführen. Bei den hierbei ablaufenden mikrobiologischen Prozessen können auch von den Mikroorganismen Enzyme an das Substrat abgegeben werden. Diese extracellulären Enzyme, z. B. Proteasen, zeigen dabei die gleiche enzymatische Aktivität wie in Lösung und bauen Peptide zu Aminosäuren ab. Diese enzymatische Aktivität bleibt sogar dann noch erhalten, wenn die zugeführte Wassermenge nicht mehr ausreichend ist, um den mikrobiologischen Prozeß aufrechtzuerhalten.

Im Unterschied hierzu betrifft die vorliegende enzymatische Reaktion nicht mikrobiologische Prozesse, bei denen Mikroorganismen mit Luft und Feuchtigkeit versorgt werden müssen, sondern enzymatische Umsetzungen ohne Mitwirkung von Mikroorganismen. Wenn erfindungsgemäß bei der enzymatischen Reaktion die Ausbeute durch Hydrolyse eines Reaktanten vermindert ist, muß natürlich zum Unterschied der Feststoff-Fermentation, bei der der Feuchtigkeitsgehalt des Substrats etwa 70 % beträgt und ein resultierender Feuchtigkeitsverlust durch feuchte Luft mit 90 - 97 % rel. Feuchte kompensiert wird, mit derart konditionierter Feuchtigkeit des durchgeleiteten Gases gearbeitet werden, daß die erfindungsgemäße enzymatische Reaktion und nicht die Hydrolyse begünstigt ist.

Da die vorliegenden Reaktionen mit einer minimalen Wassermenge auskommen, kann die Reaktion durch verschiedene Eigenarten gekennzeichnet werden. Diese sind
1. das Wasser bildet zwischen Substrat und Enzym kein flüssiges Kontinuum,
2. das Wasser kann zumindest zum Teil aus der Gasphase wirken,
3. das für die Reaktion notwendige Wasser kann der Reaktion über einen Festkörper zugesetzt werden,
4. das für die Reaktion notwendige Wasser kann entweder von einem Festkörper zu dem Substrat und/oder Enzym bzw. zwischen Substrat und Enzym durch Festkörperkontakte übertragen werden.

Punkt 1 hat den Vorteil, daß sehr geringe Wassermengen eingesetzt werden können, so daß z. B. hydrolytische Nebenreaktionen vermieden werden. Außerdem kann auf organische Lösungsmittel verzichtet werden, die gewöhnlich eingesetzt werden, um das flüssige Wasserkontinuum, das bei gewöhnlichen Enzymreaktionen verwendet wird, zu verdünnen oder zu ersetzen, um ebenfalls die Wassermenge zu reduzieren. Punkt 2 hat ebenfalls den Vorteil, daß sehr geringe Wassermengen eingesetzt werden können. Das gering vorhandene Wasser geht dabei zum Teil in die Gasphase, so daß es von dort für die Reaktion zur Verfügung steht. Das Wasser kann dabei vorteilhaft auch aus der Gasphase der Reaktion zugeführt werden. Die Punkte 3 und 4 haben den Vorteil, daß das Wasser durch Konditionieren eines Festkörpers, z. B. eines Zeoliths, oder durch Konditionieren von Substrat und/oder Enzym, der Reaktion wohldosiert zugeführt werden kann.

Für die erfindungsgemäße Reaktion können die Punkte einzeln oder in Kombination zu tragen kommen, wobei insbesondere Punkt 1 mit den anderen Punkten kombiniert sein kann.

Durch die Erfindung kann die sonst bei enzymatischen Peptidsynthesen unter Zusatz mit Wasser mischbarer oder nichtmischbarer organischer Cosolventien mögliche Schädigung des Enzyms und die damit verbundene Verminderung der katalytischen Wirksamkeit ausgeschaltet werden. Des weiteren können die die Syntheseeffektivität beeinträchtigenden hydrolytischen Nebenreaktionen an Reaktanten und Produkt durch die erfindungsgemäße Verwendung von Reaktionssystemen mit minimalem Wassergehalt wesentlich reduziert werden.

Auf Zusätze von organischen Lösungsmitteln und/oder Pufferlösungen kann meist verzichtet werden, wenn für ausreichenden Kontakt der Reaktanten und des katalytisch aktiven Enzyms gesorgt wird.

Das erfindungsgemäße Verfahren kommt insbesondere dort zum Tragen, wo höhere Wassermengen die enzymatische Reaktion negativ beeinflussen. In diesen Fällen wird erfindungsgemäß die Wassermenge im wesentlichen so weit reduziert, daß bevorzugt lediglich noch die Enzymaktivität gegeben ist. Die Nebenreaktionen lassen sich dadurch meist so weit zurückdrängen, daß sie praktisch zu vernachlässigen sind. Das Verfahren erfüllt dabei dann mindestens einen der Punkte 1 bis 4.

Bei diesen Reaktionen sind vorzugsweise alle Substrate, wenn mehrere Substrate zu einem Endprodukt reagieren, fest. Da enzymatische Hydrolysen die o. g. Probleme i. d. R. nicht haben, bezieht sich das erfindungsgemäße Verfahren insbesondere auf nichthydrolytische Reaktionen.

Da enzymatische Reaktionen, wie oben erwähnt, meist durch organische Lösungsmittel gestört werden, ist es erfindungsgemäß von Vorteil, wenn die Wassermenge so weit reduziert wird, daß auf organische Lösungsmittel verzichtet werden kann. Unter Umständen kann es jedoch erforderlich oder von Vorteil sein, wenn noch geringe Lösungsmittelmengen vorhanden sind, diese sollten, insbesondere wenn sie das vorliegende Wasser lösen können, im molaren Unterschuß zum Wasser eingesetzt werden.

Besonders bevorzugt sind bei dem erfindungsgemäßen Verfahren die Kondensationsreaktionen, da bei diesen Reaktionen eine erhöhte Wassermenge meist das Gleichgewicht so verschiebt, daß die Reaktionsausbeute beeinträchtigt wird. Unter den Kondensationsreaktionen sind Peptidsynthesen wiederum bevorzugt, da das erfindungsgemäße Verfahren insbesondere für diese besonders milde Bedingungen zur Verfügung stellt.

Ein besonders bevorzugtes Verfahren, bei dem die am Verfahren beteiligten Feststoffe durch Einleiten eines Gases aufgewirbelt werden, wird vorzugsweise in einer Vorrichtung durchgeführt, deren Merkmale Verwendungsanspruch 10 wiedergibt.

Die Vorrichtung enthält eine Kammer, die geeignet ist, die verwendeten Substrate und Enzyme aufzunehmen, wobei der Boden der Kammer gasdurchlässig sein soll aber die o. g. festen Stoffe zurückhält. Durch den Boden der Kammer kann ein Gas eingeleitet werden, wobei der Boden so ausgestaltet ist, daß bei einer bestimmten Gasströmung durch den Boden die festen Stoffe in der Kammer in eine Wirbelschicht überführt werden. Außerdem benötigt die Vorrichtung noch Mittel zum Halten der Feuchtigkeit in der Wirbelschicht bzw. im Substrat-Enzymgemisch innerhalb eines bestimmten Bereiches, so daß die enzymatische Reaktion gewährleistet ist und eventuelle Nebenreaktionen unterdrückt werden. Bei einem zu niedrigen Feuchtigkeitsgehalt kann keine enzymatische Reaktion mehr stattfinden, bei einer Erhöhung der Feuchtigkeit über einen bestimmten Wert nehmen ggf. Nebenreaktionen zu und/oder die festen Stoffe verkleben, so daß die Wirbelschicht zusammenfällt.

Vorzugsweise wird bei einer Vorrichtung der beschriebenen Art die Feuchtigkeit über das durch den gasdurchlässigen Boden eingeleitete Gas zugeführt, wobei die Feuchtigkeitsregelung in der Wirbelschicht, durch Beaufschlagen des einzuleitenden Gases mit Wasser (Dampf) erfolgt. Dieses Beaufschlagen des Gases kann z. B. durch Durchleiten des Gases durch Wasser oder eine wäßrige Salzlösung, z. B. 5 %ige Natriumhydrogencarbonat-Lösung, durch Überleiten des Gases über Salzhydrate, z. B. Calciumchloridhexahydrat oder durch sonstige bekannte Feuchtigkeitskonditioniermethoden erreicht werden.

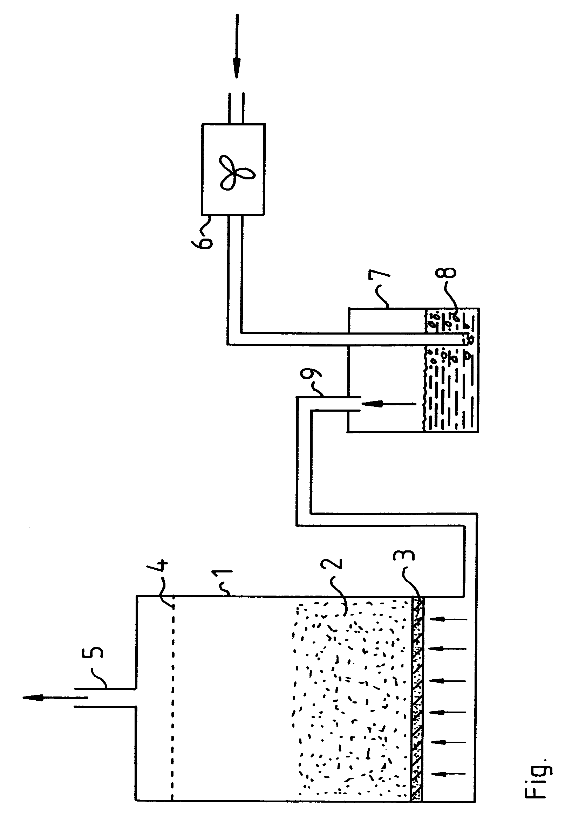

Eine solche Vorrichtung wird anhand eines Ausführungsbeispiels näher beschrieben und ist in der Figur dargestellt.

In einer Wirbelschicht 1 befindet sich ein Substrat-Enzymgemisch 2, wie es z. B. in den Beispielen 4 und 5 der Ausführungsbeispiele beschrieben ist. Die Wirbelschichtkammer 1 wird an ihrem unteren Ende von einem Boden 3 begrenzt, der gasdurchlässig ist, aber das Substrat-Enzymgemisch 2 zurückhält. Geeignete Böden dieser Art sind z. B. Glas- oder Metallfritten. Das obere Ende der Wirbelschichtkammer enthält ein Sicherungssieb 4, der zu weit aufgewirbelte Substrat-Enzymgemischfestkörperteilchen zurückhalten soll. Oberhalb des Sicherungssiebes 4 ist in der Wirbelschichtkammer 1 eine Öffnung 5 vorgesehen, durch die ein durch die Wirbelschichtkammer 1 geleitetes Gas (Pfeile) austreten kann. Als Gas eignet sich z. B. Luft oder Stickstoff.

Das Gas wird über eine steuerbare Pumpe 6 angesaugt und in eine in einem Behälter 7 befindliche Flüssigkeit 8 eingeleitet. Die Flüssigkeit 8 besteht aus einer 5 %igen Natriumhydrogencarbonat-Lösung in Wasser. Statt der Pumpe 6 kann auch eine Druckflasche mit dem Trägergas direkt an den Behälter 7 angeschlossen werden.

Über einen Auslaß 9, der genügend oberhalb der Flüssigkeit 8 angeordnet ist, verläßt das Gas den Behälter 7 und wird unter den Boden 3 in die Wirbelschichtkammer 1 eingeleitet.

Je nach Geschwindigkeit des durch den Boden 3 strömenden Gases wird in dem Substrat-Enzymgemisch, das als möglichst feinkörniges Schüttgut vorliegen soll, ein Zustand erreicht, der dem einer kochenden Flüssigkeit ähnelt. Je nach verwendeten Bedingungen (Substrat, Enzym, Wassergehalt, Temperatur etc.) wird das Substrat-Enzymgemisch 2 eine bestimmte Zeit in diesem Zustand gehalten und anschließend die Reaktion abgebrochen. Während der Reaktion wandelt sich das Substrat-Enzymgemisch zumindest teilweise in ein Produkt-Enzymgemisch um. Aus diesem Produkt-Enzymgemisch wird das Produkt - sofern gewünscht - mit üblichen Methoden isoliert.

### Ausführungsbeispiele

In den Beispielen werden die Aminosäuren und Peptide nach den international gültigen Regeln abgekürzt. Ultraschallbedingungen: 35 kHz, 50 W; Gerät USG 50 (Meßgerätewerk Ballenstedt)

### Beispiele unter Anwendung von Ultraschall

### Beispiel 1: Synthese von Z-Phe-Leu-NH₂

30 mg (0,1 mmol) Z-Phe-OH und 13 mg (0,1 mmol) Leu-NH₂ werden in ein verschließbares Glasgefäß mit 12 mm Innendurchmesser gegeben und mit einem Spatel durchmischt. Anschließend gibt man eine Mischung aus 161 mg (0,5 mmol) Na₂SO₄·10H₂O und 5 mg Thermolysin hinzu, verschließt das Glasgefäß und beschallt im Ultraschallbad bei 40°C. Nach 20 min wird die Reaktion mit 0,4 ml 5 %iger (v/v) wäßriger Trifluoressigsäure abgestoppt. Die analytische Auswertung erfolgt hier wie in den folgenden Beispielen mittels HPLC und Vergleich mit einer authentischen Probe.
Ausbeute: 87 % d Th.

### Beispiel 2: Synthese von Ac-Gly-Trp-Leu-NH₂

Wie Beispiel 1; als C-Komponente werden 30,3 mg (0,1 mmol) Ac-Gly-Trp-OH eingesetzt.
Ausbeute: 79 % d Th.

### Beispiel 3: Synthese von Z-Asp-Phe-OMe

Es werden analog Beispiel 1 26,7 mg (0,1 mmol) Z-Asp-OH, 20,3 mg (0,1 mmol) Phe-O·HC] und 28,6 mg (0,1 mmol) Na₂CO₃·10H₂O in das Glasgefäß gegeben und durchmischt. Anschließend wird die Mischung mit 12,6 mg (0,1 mmol) Na₂SO₄ und 5 mg Thermolysin versetzt. Nach 30 min Beschallung bei 40°C wird die Reaktion abgestoppt und mittels HPLC ausgewertet.
Ausbeute: 32 % d. Th.

### Beispiele unter Anwendung von Druckluft

### Beispiel 4: Synthese von Z-Ala-Phe-Leu-NH₂

38,5 mg (0,1 mmol) Z-Ala-Phe-OMe, 26,0 mg (0,2 mmol) Leu-NH₂ und 10,0 mg (0,4 µmol) Chymotrypsin werden in ein mit Frittenboden versehenes 130 mm x 20 mm Glasrohr, dessen unteres Ende zu einer Spitze ausgezogen ist, gegeben. Von unten wird Druckluft, die zuvor durch eine 5 %ige NaHCO₃-Lösung geleitet wurde, in einer solchen Weise zugeführt, daß die auf der Fritte befindlichen Feststoffe gerade aufgewirbelt werden. Zusätzlich wird das Reaktionsgefäß auf einer Schüttelapparatur bewegt. Die Reaktionszeit beträgt 3 h. Anschließend wird die Reaktionsmischung in 7 ml Methanol aufgenommen, filtriert und mittels HPLC analytisch bestimmt.
Ausbeute: 46 % d. Th.

### Beispiel 5: Synthese von Boc-Ala-Phe-Leu-NH₂

Analog Beispiel 1 werden ausgehend von 35,0 mg (0,1 mmol) Boc-Ala-Phe-OMe 22 % Boc-Ala-Phe-Leu-NH₂ erhalten.

## Patentansprüche

1. Wasserunterstützte enzymatische Reaktion zwischen einem Substrat und einem Enzym, wobei das Substrat und das Enzym in fester Phase vorliegen und das Wasser kein flüssiges Kontinuum zwischen Substrat und Enzym bildet und/oder
wobei das Substrat und das Enzym in fester Phase vorliegen und das Wasser zumindest zum Teil aus der Gasphase wirkt und/oder
wobei das Substrat und das Enzym in fester Phase vorliegen und das für die Reaktion notwendige Wasser über einen Festkörper zugesetzt wird und/oder
wobei das Substrat und das Enzym in fester Phase vorliegen und zumindest ein Teil des für die Reaktion notwendigen Wassers durch Festkörperkontakt übertragen wird,
**dadurch gekennzeichnet,**
daß die Komponenten durch Anlegen von Ultraschall innig vermischt werden.

2. Enzymatische Reaktion nach Anspruch 1,
**dadurch gekennzeichnet,**
daß die Reaktion keine Hydrolyse ist.

3. Enzymatische Reaktion nach einem der vorhergehenden Ansprüche,
**dadurch gekennzeichnet,**
daß Substrat, Enzym und Wasser während der Reaktion durch Einleiten eines Gases aufgewirbelt und innig miteinander vermischt werden.

4. Wasserunterstützte enzymatische Reaktion zwischen einem Substrat und einem Enzym zu einem Produkt, dessen Ausbeute durch Hydrolyse eines Reaktanten vermindert ist, wobei das Substrat und das Enzym in fester Phase vorliegen und das Wasser kein flüssiges Kontinuum zwischen Substrat und Enzym bildet und/oder
wobei das Substrat und das Enzym in fester Phase vorliegen und das Wasser zumindest zum Teil aus der Gasphase wirkt und/oder
wobei das Substrat und das Enzym in fester Phase vorliegen und das für die Reaktion notwendige Wasser über einen Festkörper zugesetzt wird und/oder
wobei das Substrat und das Enzym in fester Phase vorliegen, und zumindest ein Teil des für die Reaktion notwendigen Wassers durch Festkörperkontakt übertragen wird,
**dadurch gekennzeichnet,**
daß die Komponenten durch Einleiten eines Gases aufgewirbelt werden.

5. Enzymatische Reaktion nach Anspruch 4,
**dadurch gekennzeichnet,**
daß Ultraschall angelegt wird.

6. Enzymatische Reaktion nach einem der vorhergehenden Ansprüche,
**dadurch gekennzeichnet,**
daß zumindest ein Teil des für die Reaktion notwendigen Wassers mit dem Gas zugeführt wird.

7. Enzymatische Reaktion nach einem der vorhergehenden Ansprüche,
**dadurch gekennzeichnet,**
daß organische Lösungsmittel nicht oder im molaren Unterschuß zum Wasser eingesetzt werden.

8. Enzymatische Reaktion nach einem der vorhergehenden Ansprüche,
**dadurch gekennzeichnet,**
daß die Reaktion eine Kondensation ist.

9. Enzymatische Reaktion nach Anspruch 8,
**dadurch gekennzeichnet,**
daß die Reaktion eine Peptidsynthese ist.

10. Verwendung einer Vorrichtung aus
einer Wirbelschichtkammer mit einem Boden, der gasdurchlässig ist aber ein Substrat-Enzymgemisch zurückhält,
Mitteln zum Einleiten von Gas durch den gasdurchlässigen Boden, und
Mitteln zum Halten der Feuchtigkeit eines in der Wirbelschichtkammer befindlichen Substrat-Enzymgemisches innerhalb eines bestimmten Bereiches,
zur Durchführung des Verfahrens gemäß Anspruch 4 oder 5.
